# EUROPEAN PATENT APPLICATION

(11) **EP 4 814 446 A1**
(43) Date of publication of application: **30.09.2026**
(21) Application number: 26167819.7
(22) Date of filing: 25.03.2026
(51) Int. Cl.: G01F 22/00, B67D 1/00, B01F 23/237, G01F 23/00, F17C 13/02, B01F 23/236, G01N 33/14, G08B 21/18, G06V 10/82

(54) **METHOD FOR OBTAINING RESIDUAL GAS AMOUNT IN GAS CYLINDER, PROMPTING METHOD AND SODA WATER MACHINE**

(30) Priority: 25.03.2025 CN 202510356456
(71) Applicant: Zhangzhou Solex Smart Home Co., Ltd., Changtai County Zhangzhou City, Fujian Province 363900 (CN)
(72) Inventor: LU, Wei, Zhangzhou City, 363900 (CN); ZOU, Jianbo, Zhangzhou City, 363900 (CN); LIU, Jun, Zhangzhou City, 363900 (CN)
(74) Representative: Verscht, Thomas Kurt Albert

(57) **Abstract**

The present disclosure discloses a method for obtaining a residual gas amount in a gas cylinder, a method for prompting the residual gas amount in the gas cylinder and a soda water machine, the method for obtaining the residual gas amount comprises: acquiring an image of a liquid to be detected and an ambient temperature for bubble generation; identifying a current number of bubbles in the image of the liquid to be detected; acquiring a temperature coefficient representing an influence on gas concentration based on the ambient temperature for the bubble generation; and obtaining a ratio of the current number of the bubbles and a number of the bubbles when the residual gas amount is 100%, and obtaining the residual gas amount by calculation of multiplying the ratio by the temperature coefficient. In the present disclosure, the residual gas amount in the gas cylinder is obtained by acquiring an image of a liquid to be detected through an imaging device, identifying a number of bubbles in the image through a processing chip and combining a corresponding ambient temperature, and is prompted through a color ring at a water faucet, thus meeting detection requirement for the residual gas amount in the gas cylinder.

## Description

### RELATED APPLICATIONS

This application claims priority to Chinese patent application number 202510356456.X, filed on March 25, 2025. Chinese patent application number 202510356456.X is incorporated herein by reference.

### FIELD OF THE DISCLOSURE

The present disclosure relates to the technical field of preparing sparkling water, and in particular relates to a method for obtaining a residual gas amount in a gas cylinder, a method for prompting the residual gas amount in the gas cylinder and a soda water machine.

### BACKGROUND OF THE DISCLOSURE

At present, many sparkling water products on the market such as soda water machines do not judge residual gas amounts of gas cylinders, the residual gas amount is roughly estimated based on the service time in most cases, such a method is inaccurate and unscientific, and if the gas cylinder is replaced when there is still gas left inside, it is easy to cause waste; and if the gas in the gas cylinder has been used up but not replaced in time, thus resulting in a failure of the soda water machine to produce soda water, which affects user satisfaction.

At present, some sparkling water products use pressure sensors to detect gas amount in the gas cylinders, but detection with the pressure sensors requires major hardware transformation of the existing sparkling water products.

### BRIEF SUMMARY OF THE DISCLOSURE

The main object of the present disclosure is to provide a method for obtaining a residual gas amount in a gas cylinder, a prompting method and a soda water machine, which overcomes the deficiencies in the existing techniques, the residual gas amount in the gas cylinder is obtained by acquiring an image of a liquid to be detected through an imaging device, identifying a number of bubbles in the image through a processing chip and combining a corresponding ambient temperature, and is prompted through a color ring at a water faucet, thus meeting detection requirement for the residual gas amount in the gas cylinder.

The present disclosure adopts the following technical solution:

In a first aspect, a method for obtaining a residual gas amount in a gas cylinder, it comprises:

Acquiring an image of a liquid to be detected and an ambient temperature for bubble generation;

Identifying a current number of bubbles in the image of the liquid to be detected;

Acquiring a temperature coefficient representing an influence on gas concentration based on the ambient temperature for the bubble generation;

Obtaining a ratio of the current number of the bubbles and a number of the bubbles when the residual gas amount is 100%, and obtaining the residual gas amount by calculation of multiplying the ratio by the temperature coefficient.

Preferably, after acquiring the image of the liquid to be detected, the method further comprises preprocessing, specifically comprises:
Identifying a color, viscosity and presence or absence of the bubbles in the liquid to be detected;

If it is transparent, non-viscous and comprises the bubbles, proceeding to a subsequent step; otherwise, skipping execution of the subsequent step.

Preferably, before the preprocessing, the method further comprises:

Performing color correction on the acquired image of the liquid to be detected.

Preferably, after the preprocessing, the method further comprises: selecting a liquid image meeting a preset clarity as the image of the liquid to be detected.

Preferably, the temperature coefficient is obtained by looking up a chart or by a fitted equation; and the chart or the equation represents a variation relationship of the temperature coefficient with the ambient temperature for the bubble generation.

Preferably, when the ambient temperature is not higher than a preset value in degrees Celsius, and the temperature coefficient is 1; and when the ambient temperature is higher than the preset value in degrees Celsius, the temperature coefficient is negatively correlated with the ambient temperature.

In a second aspect, A method for prompting a residual gas amount in a gas cylinder, it is based on the method for obtaining the residual gas amount in the gas cylinder, the method comprises:
Prompting the residual gas amount through a color change of a light ring at a water faucet based on a range of the residual gas amount.

In a third aspect, a soda water machine, comprises a shell and a water faucet, a carbonation tank, a refrigeration system connected with the carbonation tank, a water inlet path unit and a water outlet path unit are installed in the shell, the carbonation tank comprises an external interface for externally connecting a carbon dioxide gas cylinder, a carbonation chamber and a water chamber are disposed in the carbonation tank; it further comprises a processing chip and an imaging device installed in the carbonation chamber; the imaging device acquires an image of a liquid to be detected and sends to the processing chip; and the processing chip executes the method for obtaining the residual gas amount in the gas cylinder.

Preferably, the soda water machine further comprises a light ring disposed at a handle ring of the water faucet; and a color of the light ring changes with the residual gas amount in the gas cylinder.

In a fourth aspect, a method for obtaining a residual gas amount in a gas cylinder, it comprises:

Acquiring an image of a liquid to be detected and ambient temperature for bubble generation;

Identifying a current number of bubbles in the image of the liquid to be detected;

Inputting the current number of the bubbles and the ambient temperature for the bubble generation into a trained neural network to obtain the residual gas amount.

Preferably, after acquiring the image of the liquid to be detected, the method further comprises preprocessing, specifically comprises:
Identifying a color, viscosity and presence or absence of the bubbles in the liquid to be detected;

If it is transparent, non-viscous and comprises the bubbles, proceeding to a subsequent step; otherwise, skipping execution of the subsequent step.

In a fifth aspect, a method for prompting a residual gas amount in a gas cylinder, it is based on the method for obtaining the residual gas amount in the gas cylinder, the method comprises:

Prompting the residual gas amount through a color change of a light ring at a water faucet based on a range of the residual gas amount.

In a sixth aspect, a soda water machine, comprises a shell and a water faucet, a carbonation tank, a refrigeration system connected with the carbonation tank, a water inlet path unit and a water outlet path unit are installed in the shell, the carbonation tank comprises an external interface for externally connecting a carbon dioxide gas cylinder, and a carbonation chamber and a water chamber are disposed in the carbonation tank; it further comprises a processing chip and an imaging device installed in the carbonation chamber; the imaging device acquires an image of a liquid to be detected and sends to the processing chip; and the processing chip executes the method for obtaining the residual gas amount in the gas cylinder.

Preferably, the soda water machine further comprises a light ring disposed at a handle ring of the water faucet; and a color of the light ring changes with the residual gas amount in the gas cylinder.

Compared with the existing techniques, The technical solution has the following advantages:
(1) The method for obtaining the residual gas amount in the gas cylinder of the present disclosure can obtain the residual gas amount in the gas cylinder only by acquiring the number of the bubbles in the image of the liquid to be detected through image analysis and acquiring the ambient temperature for the bubble generation through a temperature acquisition device; and the residual gas amount in the gas cylinder is obtained based on the number of the bubbles and the ambient temperature, which can be obtained by calculating according to data in the chart/table obtained through experiments or obtained according to a neural network model pre-trained with experimental data, and the method is simple and easy to implement;
(2) For products with multiple liquid outputs, the method for obtaining the residual gas amount in the gas cylinder of the present disclosure judges a liquid type according to liquid color, bubbles and viscosity before identifying the number of the bubbles, and only identifies the number of the bubbles for a liquid generated with gas mixed therein, which can reduce analysis of irrelevant data and calculation amount;
(3) The method for prompting the residual gas amount in the gas cylinder of the present disclosure prompts the residual gas amount through the color change of the light ring at the handle ring of the water faucet, so that a user can intuitively perceive a change of the residual gas amount in the gas cylinder; and
(4) The soda water machine of the present disclosure can obtain the residual gas amount in the gas cylinder only by adding the imaging device in the existing carbonation chamber, and can prompt the residual gas amount only by adding a color ring at the water faucet, with low hardware transformation cost.

The aforementioned description is only a summary of the technical solutions of the present disclosure, in order to understand the technical means of the present disclosure more clearly, the present disclosure can be implemented according to the content of the specification, and in order to make the aforementioned and other objects, features and advantages of the present disclosure more explicit and understandable, the specific implementations of the present disclosure are listed below.

Those of skill in the art will further understand the aforementioned and other objects, advantages and features of the present disclosure according to the detailed description of the specific embodiments of the present disclosure based on the following description in combination with the accompanying drawings.

### BRIEF DESCRIPTION OF THE DRAWINGS

FIG. 1 illustrates a flow schematic diagram of a method for obtaining a residual gas amount in a gas cylinder in Embodiment 1 of the present disclosure;
FIG. 2 illustrates a diagrammatic view of an image of a liquid to be detected containing bubbles obtained in Embodiment 1 of the present disclosure;
FIG. 3 illustrates a diagrammatic view of the residual gas amount affected by temperature in Embodiment 1 of the present disclosure;
FIG. 4 illustrate a diagrammatic view of color indication of a color ring in Embodiment 1 of the present disclosure; and wherein (a) illustrates a diagrammatic view of a water faucet as a whole; and (b) illustrates a view of the light ring at a handle ring of the water faucet;
FIG. 5 illustrates a structural diagrammatic view of a soda water machine in Embodiment 1 of the present disclosure; and
FIG. 6 is a flow schematic chart of a method for obtaining a residual gas amount in a gas cylinder in Embodiment 2 of the present disclosure.

### DETAILED DESCRIPTION OF THE EMBODIMENTS

The technical solutions in the embodiments of the present disclosure will be clearly and completely described below in combination with the accompanying drawings in the embodiments of the present disclosure; and it is obvious that the described embodiments are only some embodiments rather than all embodiments of the present disclosure, all other embodiments shall fall within the protection scope of the present disclosure provided that they are obtained by those of ordinary skill in the art without creative works based on the embodiments of the present disclosure.

In the description of the present disclosure, it should be noted that the terms "comprise", "include" or any other variants thereof are intended to cover non-exclusive inclusion, so that a process, a method, a product or a device including a series of elements not only includes those elements, but also includes other elements not explicitly listed, or also includes the inherent elements of the process, the method, the product or the device. Unless further restrictions are specified, an element limited by a sentence "comprising a..." does not exclude an existence of other identical elements in the process, the method, the product or the device including the element.

In the description of the present disclosure, it should be further noted that unless otherwise clearly specified and limited, the terms of "installed", "disposed", "sleeved/socketed", "connected" should be construed broadly, for example, "connected" can be a fixed connection, a detachable connection, or an integral connection, a mechanical connection, an electrical connection, a direct connection, an indirect connection through an intermediate medium, or an internal communication between two elements, and for those of ordinary skill in the art, the specific meanings of the terms in the present disclosure can be construed according to specific circumstances.

In the description of the present disclosure, it should be noted that unless otherwise clearly specified and limited, step identifiers such as S101, S102, S103 and the like are only used for convenient description and do not indicate an execution order, and a corresponding execution order can be adjusted.

### Embodiment 1

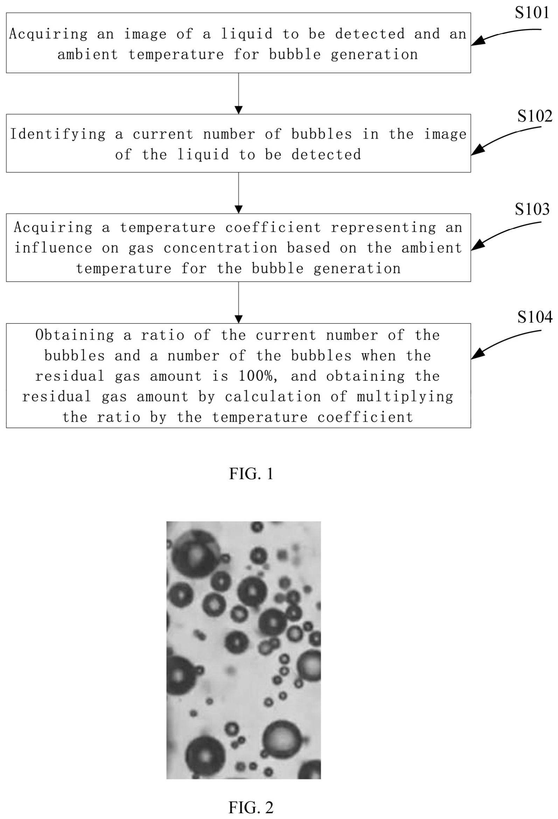

As shown in FIG. 1, a method for obtaining a residual gas amount in a gas cylinder in this embodiment comprises:
S101, acquiring an image of a liquid to be detected and an ambient temperature for bubble generation;
S102, identifying a current number of bubbles in the image of the liquid to be detected;
S103, acquiring a temperature coefficient representing an influence on gas concentration based on the ambient temperature for the bubble generation; and
S104, obtaining a ratio of the current number of the bubbles and a number of the bubbles when the residual gas amount is 100%, and obtaining the residual gas amount by calculation of multiplying the ratio by the temperature coefficient.

In this embodiment, the method for obtaining the residual gas amount in the gas cylinder is achieved by a processing chip, and the image of the liquid to be detected is acquired by an imaging device such as a camera and then sent to the processing chip.

Specifically, a 500-1000 times high-definition high-speed camera can be adopted to acquire the image of the liquid in a carbonation chamber, and the image of the liquid is sent to the processing chip for analysis and processing, the residual gas amount is obtained by the calculation, and the residual gas amount is fed back to prompt a user.

In the S101, after the image of the liquid to be detected is acquired, the image needs to be preprocessed to identify transparent sparkling water. Specifically, three features of color, bubbles, viscosity of the liquid are matched with features of liquid types prestored in a database to obtain a type of the liquid to be detected, if the liquid to be detected is not the transparent sparkling water, S102 is not executed, so as to reduce unnecessary processing and prevent erroneous data in the counted number of the bubbles. A corresponding relationship between the liquid types and the color, the bubbles, the viscosity is shown in Table 1 below.

**Table 1**

| Liquid type | Bubbles | Viscosity | Color | Judge whether to execute to a subsequent step |
|---|---|---|---|---|
| Soda water | Yes | No | Transparent | YES |
| Low-concentration soda water | Yes | No | Transparent | YES |
| Pure water | No | No | Transparent | NO |
| Syrup water | Yes | Yes | Transparent | NO |
| Fanta | Yes | No | Opaque | NO |
| Cola | Yes | No | Opaque | NO |

Further, in order to avoid the color from being affected by ambient light, it is necessary to perform color correction on the acquired image of the liquid to be detected before preprocessing. Specifically, parameters of the ambient light are obtained by photographing a color calibration board using the camera, and the color of the image is adjusted by using a color correction algorithm through these parameters to improve color reproduction of imaging.

In practice, matching correction can be performed by extracting changes of the liquid generated under the ambient light. In this embodiment, various liquid images are captured in advance and color analysis is performed. For example, a color of the pure water under the ambient light, a color of the soda water under light, a color of the syrup water under light and the like, by learning these colors, a liquid part in the image of the liquid to be detected can be identified through matching.

Further, if the image of the liquid to be detected complies with an image in the preset liquid types, the camera is controlled to continue acquiring, specifically, the camera is controlled to take continuous photos, and one with good clarity is selected to perform statistics of the number of the bubbles in S102. FIG. 2 illustrates a diagrammatic view of the image of the liquid to be detected containing the bubbles.

In the S102, the method for identifying the current number of the bubbles in the image of the liquid to be detected can adopt any method in the existing techniques. One implementable method can be achieved through a machine learning method, sufficient images of the bubbles are obtained in advance, the images of the bubbles are input into a machine learning model for training, and the model can be used for identifying the bubbles and the number of bubbles in the image in the method of the present disclosure after training.

In the S103, the temperature coefficient representing the influence on the gas concentration is acquired based on the ambient temperature for the bubble generation, and the specific implementation is as follows.

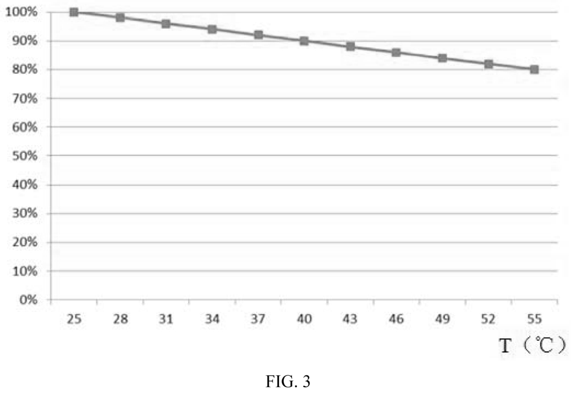

Specifically, a gas concentration is defined as 100% when the gas cylinder is in a first use (with the temperature within 25 °C), and a total number of the bubbles in a water body is acquired during the first use. During subsequent use, the current number of the bubbles in the water body is detected in real time, and a temperature coefficient (gas concentration) at a current temperature is looked up according to a correspondence chart of the gas concentration and the temperature shown in FIG. 3, and a calculation formula of the final residual gas amount in the gas cylinder is as follows: $C = \frac{{W}_{n}}{{W}_{total}} ⋅ {T}_{coeff .} ⋅ 100 %$

Wherein, C is the residual gas amount in the gas cylinder, W*ₙ* is the current number of the bubbles in the image of the liquid to be detected, W*ₜₒₜₐₗ* is the total number of the bubbles in the water body when the gas concentration is 100%, T*_{coeff}.* is the temperature coefficient representing influence on the gas concentration, T*_{coeff.}* can be looked up in the chart, and in other embodiments, the chart can also be looked up by fitting into a curve, which is not specifically limited in this embodiment.

For example, in the first use: *Wₜₒₜₐₗ* is 1000; and T*_{coeff.}* is 100%=1 (the influence from the temperature is not calculated in the first use, which is defaulted to 100%).

After being used for half a year, W*ₙ* is 330; and the temperature is 38 °C, and T*_{coeff.}* can be then obtained as 90%=0.9 by looking up in the following chart;

Thus, after being used for half a year, the residual gas amount in the gas cylinder can be calculated as: $330 / 1000 * 0.9 * 100 % = 29.7 % .$

Further, this embodiment also discloses a method for prompting the residual gas amount in the gas cylinder based on the method for obtaining the residual gas amount in the gas cylinder, which comprises:
The residual gas amount is promoted through a color change of a light ring at a water faucet according to a range of the residual gas amount.

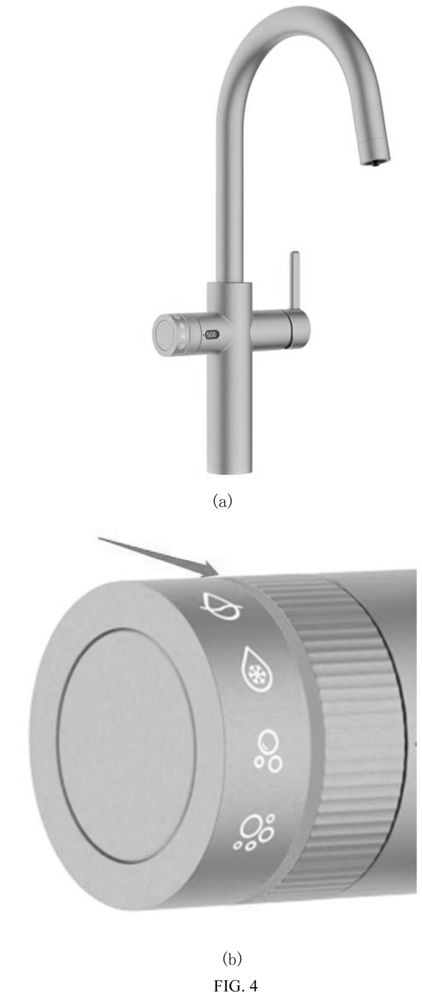

As shown in Fig. 4, in an embodiment, the color change of the light ring can be divided into 6 grades, correspondingly, when the residual gas amount is 100%-80%, the light ring displays white; when the residual gas amount is 79%-60%, the light ring displays cyan; when the residual gas amount is 59%-40%, the light ring displays blue; when the residual gas amount is 39%-20%, the light ring displays orange; when the residual gas amount is 19%-10%, the light ring displays red; and when the residual gas amount is below 10%, the light ring blinks red. In this way, the user can intuitively perceive a change of the residual gas amount in the gas cylinder, and perform corresponding treatment as needed, and it is also convenient for the user to locate the reason why the sparkling water cannot be produced.

It should be noted that the method for prompting the residual gas amount can also be implemented by voice prompt, interface prompt and the like, which are not listed one by one in this embodiment.

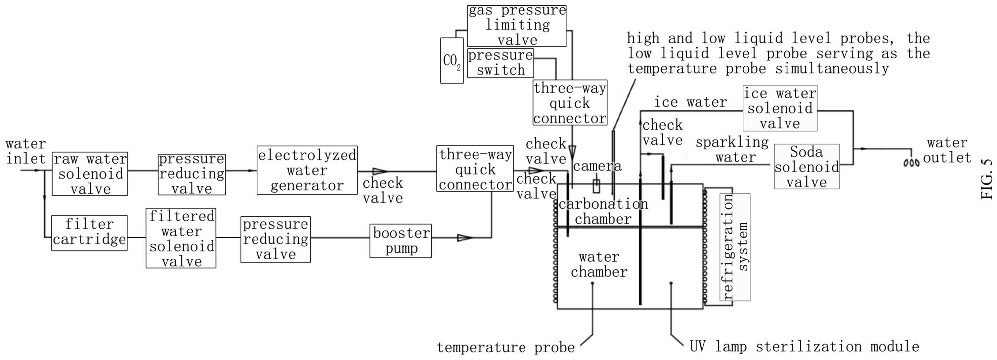

Further, as shown in Fig. 5, this embodiment also discloses a soda water machine, which comprises a shell and a water faucet, a carbonation tank, a refrigeration system connected with the carbonation tank, a water inlet path unit and a water outlet path unit are installed in the shell, the carbonation tank comprises an external interface for externally connecting carbon dioxide gas, and a carbonation chamber and a water chamber are disposed in the carbonation tank; it further comprises a processing chip, a camera installed in the carbonation chamber and a temperature probe disposed in the water chamber; the camera acquires the image of the liquid to be detected and sends to the processing chip (not shown in the drawings); the temperature probe is configured to acquire the ambient temperature for bubble generation and send to the processing chip; and the processing chip executes the method for obtaining the residual gas amount in the gas cylinder.

In this embodiment, the soda water machine further comprises the light ring disposed at the handle ring of the water faucet (as shown in Fig. 4); and a color of the light ring changes with the residual gas amount in the gas cylinder, and the specific change process refers to the aforementioned implement.

### Embodiment 2

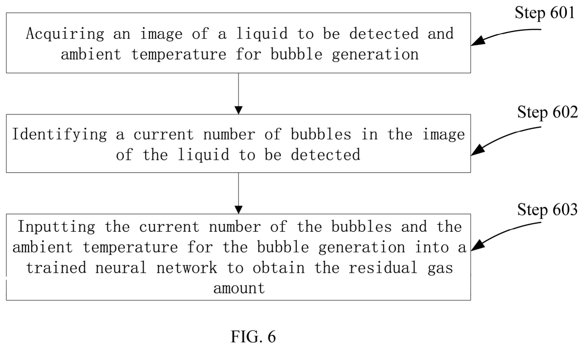

As shown in Fig. 6, a method for obtaining a residual gas amount in a gas cylinder in this embodiment comprises:
S601, acquiring an image of a liquid to be detected and ambient temperature for bubble generation;
S602, identifying a current number of bubbles in the image of the liquid to be detected; and
S603, inputting the current number of the bubbles and the ambient temperature for the bubble generation into a trained neural network to obtain the residual gas amount.

This embodiment differs from Embodiment 1 in that the residual gas amount is obtained through the neural network.

Specifically, multiple groups of known data are sent to the neural network for training, the data comprises the number of the bubbles in a water body, a value of the temperature and the residual amount of carbon dioxide, and the residual amount of the carbon dioxide can be obtained through methods such as a weighing method and measurement by a pressure gauge, and after the neural network is trained, the residual amount of the carbon dioxide can be obtained by inputting the number of the bubbles in the water body and the value of the temperature.

The implementation of the S601 and the S602 in this embodiment is the same as that in Embodiment 1, and will not be repeated herein.

Correspondingly, this embodiment further discloses a method for prompting the residual gas amount in the gas cylinder based on the method for obtaining the residual gas amount in the gas cylinder, which comprises:

The residual gas amount is promoted through a color change of a light ring at a water faucet according to a range of the residual gas amount.

Further, this embodiment also discloses a soda water machine, which comprises a shell and a water faucet, a carbonation tank, a refrigeration system connected with the carbonation tank, a water inlet path unit and a water outlet path unit are installed in the shell, the carbonation tank comprises an external interface for externally connecting carbon dioxide gas, and a carbonation chamber and a water chamber are disposed in the carbonation tank; it is characterized in that it further comprises a processing chip and a camera installed in the carbonation chamber; the camera acquires the image of the liquid to be detected and sends to the processing chip; and the processing chip executes the method for obtaining the residual gas amount in the gas cylinder.

The soda water machine further comprises the light ring disposed at a handle ring of the water faucet; and a color of the light ring changes with the residual gas amount in the gas cylinder.

The specific implementation of the method for prompting the residual gas amount in the gas cylinder and the soda water machine in this embodiment is the same as that in Embodiment 1, and will not be repeated herein.

The present invention may be summarized as follows: The present disclosure discloses a method for obtaining a residual gas amount in a gas cylinder, a method for prompting the residual gas amount in the gas cylinder and a soda water machine, the method for obtaining the residual gas amount comprises: acquiring an image of a liquid to be detected and an ambient temperature for bubble generation; identifying a current number of bubbles in the image of the liquid to be detected; acquiring a temperature coefficient representing an influence on gas concentration based on the ambient temperature for the bubble generation; and obtaining a ratio of the current number of the bubbles and a number of the bubbles when the residual gas amount is 100%, and obtaining the residual gas amount by calculation of multiplying the ratio by the temperature coefficient. In the present disclosure, the residual gas amount in the gas cylinder is obtained by acquiring an image of a liquid to be detected through an imaging device, identifying a number of bubbles in the image through a processing chip and combining a corresponding ambient temperature, and is prompted through a color ring at a water faucet, thus meeting detection requirement for the residual gas amount in the gas cylinder.

The aforementioned description is merely preferred specific implementations of the present disclosure; however, the protection scope of the present disclosure is not limited thereto. Equivalent replacements or changes fall within the protection scope of the present disclosure provided that they are made according to the technical solution of the present disclosure and its improved conception within the technical scope disclosed by the present disclosure by any person of skill in the art.

## Claims

1. A method for obtaining a residual gas amount in a gas cylinder, **characterized in that**, it comprises:
acquiring an image of a liquid to be detected and an ambient temperature for bubble generation;
identifying a current number of bubbles in the image of the liquid to be detected;
acquiring a temperature coefficient representing an influence on gas concentration based on the ambient temperature for the bubble generation; and
obtaining a ratio of the current number of the bubbles and a number of the bubbles when the residual gas amount is 100%, and obtaining the residual gas amount by calculation of multiplying the ratio by the temperature coefficient.

2. The method for obtaining the residual gas amount in the gas cylinder according to claim 1, **characterized in that**: after acquiring the image of the liquid to be detected, the method further comprises preprocessing, specifically comprises:
identifying a color, viscosity and presence or absence of the bubbles in the liquid to be detected; and
if it is transparent, non-viscous and comprises the bubbles, proceeding to a subsequent step; otherwise, skipping execution of the subsequent step.

3. The method for obtaining the residual gas amount in the gas cylinder according to claim 2, **characterized in that**: before the preprocessing, the method further comprises:
performing color correction on the acquired image of the liquid to be detected.

4. The method for obtaining the residual gas amount in the gas cylinder according to claim 2 and/or 3, **characterized in that**: after the preprocessing, the method further comprises: selecting a liquid image meeting a preset clarity as the image of the liquid to be detected.

5. The method for obtaining the residual gas amount in the gas cylinder according to any one or more of claims 1 to 4, **characterized in that**: the temperature coefficient is obtained by looking up a chart or by a fitted equation; and the chart or the equation represents a variation relationship of the temperature coefficient with the ambient temperature for the bubble generation.

6. The method for obtaining the residual gas amount in the gas cylinder according to any one or more of claims 1 to 5, **characterized in that**: when the ambient temperature is not higher than a preset value in degrees Celsius, and the temperature coefficient is 1; and when the ambient temperature is higher than the preset value in degrees Celsius, the temperature coefficient is negatively correlated with the ambient temperature.

7. A method for prompting a residual gas amount in a gas cylinder, **characterized in that**, it is based on the method for obtaining the residual gas amount in the gas cylinder according to any one or more of claims 1-6, the method comprises:
prompting the residual gas amount through a color change of a light ring at a water faucet based on a range of the residual gas amount.

8. A soda water machine, it comprises a shell and a water faucet, a carbonation tank, a refrigeration system connected with the carbonation tank, a water inlet path unit and a water outlet path unit are installed in the shell, the carbonation tank comprises an external interface for externally connecting a carbon dioxide gas cylinder, and a carbonation chamber and a water chamber are disposed in the carbonation tank; **characterized in that**: it further comprises a processing chip and an imaging device installed in the carbonation chamber; the imaging device acquires an image of a liquid to be detected and sends to the processing chip; and the processing chip executes the method for obtaining the residual gas amount in the gas cylinder according to any one or more of claims 1-6.

9. The soda water machine according to claim 8, **characterized in that**, it further comprises a light ring disposed at a handle ring of the water faucet; and a color of the light ring changes with the residual gas amount in the gas cylinder.

10. A method for obtaining a residual gas amount in a gas cylinder, **characterized in that**, it comprises:
acquiring an image of a liquid to be detected and ambient temperature for bubble generation;
identifying a current number of bubbles in the image of the liquid to be detected; and
inputting the current number of the bubbles and the ambient temperature for the bubble generation into a trained neural network to obtain the residual gas amount.

11. The method for obtaining the residual gas amount in the gas cylinder according to claim 10, **characterized in that**, after acquiring the image of the liquid to be detected, the method further comprises preprocessing, specifically comprises:
identifying a color, viscosity and presence or absence of the bubbles in the liquid to be detected; and
if it is transparent, non-viscous and comprises the bubbles, proceeding to a subsequent step; otherwise, skipping execution of the subsequent step.

12. A method for prompting a residual gas amount in a gas cylinder, **characterized in that**, it is based on the method for obtaining the residual gas amount in the gas cylinder according to any one or more of claims 10-11, the method comprises:
prompting the residual gas amount through a color change of a light ring at a water faucet based on a range of the residual gas amount.

13. A soda water machine, it comprises a shell and a water faucet, a carbonation tank, a refrigeration system connected with the carbonation tank, a water inlet path unit and a water outlet path unit are installed in the shell, the carbonation tank comprises an external interface for externally connecting a carbon dioxide gas cylinder, and a carbonation chamber and a water chamber are disposed in the carbonation tank; **characterized in that**: it further comprises a processing chip and an imaging device installed in the carbonation chamber; the imaging device acquires an image of a liquid to be detected and sends to the processing chip; and the processing chip executes the method for obtaining the residual gas amount in the gas cylinder according to any one or more of claims 10-11.

14. The soda water machine according to claim 13, **characterized in that**, it further comprises a light ring disposed at a handle ring of the water faucet; and a color of the light ring changes with the residual gas amount in the gas cylinder.
